# EUROPEAN PATENT APPLICATION

(11) **EP 1 598 424 A1**
(43) Date of publication of application: **23.11.2005**
(21) Application number: 04706847.3
(22) Date of filing: 30.01.2004
(51) Int. Cl.: C12N 15/86

(54) **PARAMYXOVIRUS VECTOR ENCODING RIBOZYME AND UTILIZATION THEREOF**

(30) Priority: 31.01.2003 JP 2003023313
(71) Applicant: Dnavec Research Inc., Tsukuba-shi, Ibaraki 305-0856 (JP)
(72) Inventor: TOKUSUMI, Tsuyoshi, c/o Dnavec Research Inc., Tsukuba-shi, Ibaraki 305-0856 (JP); BAN, Hiroshi, c/o Dnavec Research Inc., Tsukuba-shi, Ibaraki 305-0856 (JP); IIDA, Akihiro, c/o Dnavec Research Inc., Tsukuba-shi, Ibaraki 305-0856 (JP); HASEGAWA, Mamoru, c/o Dnavec Research Inc., Tsukuba-shi, Ibaraki 305-0856 (JP)
(74) Representative: Vossius & Partner
(86) International application number: PCT/JP2004/000944
(87) International publication number: WO 2004/067752

(57) **Abstract**

The present invention provides paramyxoviral vectors encoding active ribozymes. The vectors of the present invention are suitable as vectors for gene therapy to express desired ribozymes in a broad range of tissues *in vivo* or *ex vivo.* The vectors of the present invention can be used in gene therapy for cancers and other diseases.

SEQUENCE LISTING

<110>  DNAVEC RESEARCH INC.

<120>  PARAMYXOVIRUS VECTOR ENCODING RIBOZYME AND UTILIZATION THEREOF

<130>  D3-A0207P

<140>
<141>

<150> JP   2003-023313
<151> 2003-01-31

<160> 38

<170> PatentIn Ver. 2.1

<210> 1
<211> 10
<212> RNA
<213> Artificial Sequence

<220>
<223> Description of Artificial Sequence: an example of
      Sendai virus S sequence (w= a or c; v= a or c or g)

<400> 1
cwuuvwcccu                                                        10

<210> 2
<211> 10
<212> RNA
<213> Artificial Sequence

<220>
<223> Description of Artificial Sequence: an example of
      Sendai virus S sequence

<400> 2
cuuugacccu                                                            10


<210> 3
<211> 10
<212> RNA
<213> Artificial Sequence

<220>
<223> Description of Artificial Sequence: an example of
      Sendai virus S sequence

<400> 3
cauucacccu                                                            10


<210> 4
<211> 10
<212> RNA
<213> Artificial Sequence

<220>
<223> Description of Artificial Sequence: an example of
      Sendai virus S sequence

<400> 4
cuuucacccu                                                            10


<210> 5

<211> 10
<212> DNA
<213> Artificial Sequence

<220>
<223> Description of Artificial Sequence: an example of
      Sendai virus S sequence

<400> 5
agggtcaaag                                                            10


<210> 6
<211> 10
<212> DNA
<213> Artificial Sequence

<220>
<223> Description of Artificial Sequence: an example of
      Sendai virus S sequence

<400> 6
agggtgaatg                                                            10


<210> 7
<211> 10
<212> DNA
<213> Artificial Sequence

<220>
<223> Description of Artificial Sequence: an example of
      Sendai virus S sequence

<400> 7
agggtgaaag                                                            10

<210> 8
<211> 9
<212> RNA
<213> Artificial Sequence

<220>
<223> Description of Artificial Sequence: an example of
      Sendai virus E sequence

<400> 8
uuuuucuua                                                              9


<210> 9
<211> 9
<212> DNA
<213> Artificial Sequence

<220>
<223> Description of Artificial Sequence: an example of
      Sendai virus E sequence

<400> 9
taagaaaaa                                                              9


<210> 10
<211> 10
<212> DNA
<213> Artificial Sequence

<220>
<223> Description of Artificial Sequence: an example of
      Sendai virus S sequence

<400> 10
ctttcaccct                                                              10

<210> 11
<211> 15
<212> DNA
<213> Artificial Sequence

<220>
<223> Description of Artificial Sequence: an example of
      Sendai virus E sequence

<400> 11
tttttcttac tacgg                                                        15

<210> 12
<211> 18
<212> DNA
<213> Artificial Sequence

<220>
<223> Description of Artificial Sequence: an
      artificially synthesyzed linker sequence

<400> 12
atgcatgccg gcagatga                                                     18

<210> 13
<211> 18
<212> DNA
<213> Artificial Sequence

<220>
<223> Description of Artificial Sequence: an
      artificially synthesyzed primer used for PCR

<400> 13
gttgagtact gcaagagc                                                    18


<210> 14
<211> 42
<212> DNA
<213> Artificial Sequence

<220>
<223> Description of Artificial Sequence: an
      artificially synthesyzed primer used for PCR

<400> 14
tttgccggca tgcatgtttc ccaaggggag agttttgcaa cc                         42


<210> 15
<211> 18
<212> DNA
<213> Artificial Sequence

<220>
<223> Description of Artificial Sequence: an
      artificially synthesyzed primer used for PCR

<400> 15
atgcatgccg gcagatga                                                    18


<210> 16
<211> 21

<212> DNA
<213> Artificial Sequence

<220>
<223> Description of Artificial Sequence: an
      artificially synthesyzed primer used for PCR

<400> 16
tgggtgaatg agagaatcag c                                                21


<210> 17
<211> 44
<212> DNA
<213> Artificial Sequence

<220>
<223> Description of Artificial Sequence: an
      oligonucleotide used for construction of a ribozyme

<400> 17
ctagctacgc cctgatgagt ccgtgaggac gaaacagctg gtac                       44


<210> 18
<211> 36
<212> DNA
<213> Artificial Sequence

<220>
<223> Description of Artificial Sequence: an
      oligonucleotide used for construction of a ribozyme

<400> 18
cagctgtttc gtcctcacgg actcatcagg gcgtag                                36

<210> 19
<211> 47
<212> DNA
<213> Artificial Sequence

<220>
<223> Description of Artificial Sequence: an
      oligonucleotide used for construction of a ribozyme

<400> 19
tgcctacgcc ctgatgagtc cgtgaggacg aaacagctcc aactagg                47

<210> 20
<211> 48
<212> DNA
<213> Artificial Sequence

<220>
<223> Description of Artificial Sequence: an
      oligonucleotide used for construction of a ribozyme

<400> 20
ctagttggag ctgtttcgtc ctcacggact catcagggcg taggcagg               48

<210> 21
<211> 68
<212> DNA
<213> Artificial Sequence

<220>
<223> Description of Artificial Sequence: an
      oligonucleotide used for construction of a ribozyme

<400> 21
ctagcccact cttgcctacg ccctgatgag tccgtgagga cgaaacagct ccaactacca 60
caaggtac 68

<210> 22
<211> 60
<212> DNA
<213> Artificial Sequence

<220>
<223> Description of Artificial Sequence: an
      oligonucleotide used for construction of a ribozyme

<400> 22
cttgtggtag ttggagctgt ttcgtcctca cggactcatc agggcgtagg caagagtggg 60

<210> 23
<211> 80
<212> DNA
<213> Artificial Sequence

<220>
<223> Description of Artificial Sequence: an
      oligonucleotide used for construction of a ribozyme

<400> 23
ctagcctcaa ggcactcttg cctacgccct gatgagtccg tgaggacgaa acagctccaa 60
ctaccacaag tttatggtac 80

<210> 24
<211> 72
<212> DNA
<213> Artificial Sequence

<220>
<223> Description of Artificial Sequence: an
      oligonucleotide used for construction of a ribozyme

<400> 24
cataaacttg tggtagttgg agctgtttcg tcctcacgga ctcatcaggg cgtaggcaag 60
agtgccttga gg                                                      72

<210> 25
<211> 66
<212> DNA
<213> Artificial Sequence

<220>
<223> Description of Artificial Sequence: an
      oligonucleotide used for construction of pAG270

<400> 25
ggccgcgcta gcttgctcga gcaaggcgcg cctccgtaag aaaaacttag ggtgaaagtt 60
catcgc                                                             66

<210> 26
<211> 66
<212> DNA
<213> Artificial Sequence

<220>
<223> Description of Artificial Sequence: an
      oligonucleotide used for construction of pAG270

<400> 26
ggccgcgatg aactttcacc ctaagttttt cttacggagg cgcgccttgc tcgagcaagc 60
tagcgc                                                             66

<210> 27
<211> 22
<212> DNA
<213> Artificial Sequence

<220>
<223> Description of Artificial Sequence: an
      artificilally synthesized primer used for PCR

<400> 27
atgagggacc agtacatgag ga                                              22


<210> 28
<211> 30
<212> DNA
<213> Artificial Sequence

<220>
<223> Description of Artificial Sequence: an
      artificilally synthesized primer used for PCR

<400> 28
gtcgagaata tccaagagac aggtttctcc                                      30


<210> 29
<211> 72
<212> DNA
<213> Artificial Sequence

<220>
<223> Description of Artificial Sequence: an
      artificially synthesized sequence comprising

multiple cloning sites and an EIS sequence of
Sendai virus

<400> 29
ggccgcactc gagttcgcta gcttggcgcg ccggtacctc cgtaagaaaa acttagggtg 60
aaagttcatc gc 72

<210> 30
<211> 35
<212> DNA
<213> Artificial Sequence

<220>
<223> Description of Artificial Sequence: sequence
around Not I site of pSeV(+)PM

<400> 30
agggtgaaag aaatttcacc taagcggccg caatg 35

<210> 31
<211> 60
<212> RNA
<213> Homo sapiens

<220>
<221> CDS
<222> (1)..(60)

<400> 31
aug acu gaa uau aaa cuu gug gua guu gga gcu guu ggc gua ggc aag 48
Met Thr Glu Tyr Lys Leu Val Val Val Gly Ala Val Gly Val Gly Lys
1               5                   10                  15

agu gcc uug acg 60

Ser Ala Leu Thr
            20

<210> 32
<211> 20
<212> PRT
<213> Homo sapiens

<400> 32
Met Thr Glu Tyr Lys Leu Val Val Val Gly Ala Val Gly Val Gly Lys
  1               5                  10                  15

Ser Ala Leu Thr
            20

<210> 33
<211> 46
<212> RNA
<213> Artificial Sequence

<220>
<223> Description of Artificial Sequence: a ribozyme
      KRZb which cleaves K-ras mRNA

<400> 33
ugccuacgcc cugaugaguc cgugaggacg aaacagcucc aacuac                46

<210> 34
<211> 22
<212> DNA
<213> Artificial Sequence

<220>
<223> Description of Artificial Sequence: an EIS
      sequence of Sendai virus

<400> 34
taagaaaaac ttagggtcaa ag                                                22

<210> 35
<211> 24
<212> DNA
<213> Artificial Sequence

<220>
<223> Description of Artificial Sequence: flanking
      sequence around a ribozyme encoded in a vector

<400> 35
gcggccgcac tcgagttcgc tagc                                              24

<210> 36
<211> 46
<212> DNA
<213> Artificial Sequence

<220>
<223> Description of Artificial Sequence: flanking
      sequence around a ribozyme encoded in a vector

<400> 36
ggtacctccg taagaaaaac ttagggtcaa agttcatcgc ggccgc                      46

<210> 37
<211> 35

<212> DNA
<213> Artificial Sequence

<220>
<223> Description of Artificial Sequence: sequence
      around Not I site of pSeV(+)PM

<400> 37
agggtgaaag aaatttcacc taagcggccg caatg                                35

<210> 38
<211> 24
<212> RNA
<213> Artificial Sequence

<220>
<223> Description of Artificial Sequence: sequence of a
      hammerhead ribozyme (n= g or a or u or c)

<400> 38
cugangannn nnnnnnnnng aaan                                            24

## Description

### Technical Field

The present invention relates to paramyxoviral vectors encoding RNAs with catalytic activity, and uses thereof.

### Background Art

Low-molecular-weight RNAs with catalytic activity, such as ribozymes, have been used to suppress gene expression in eukaryotes, and have recently attracted attention for their application to human gene therapy. Hammerhead ribozymes are mainly active in the cytoplasm, and thus efficient transport to the cytoplasm is the key to success (Koseki, S. *et al.,* J Virol 73(3), 1868-77 (1999); Kuwabara, T. *et al.,* Proc Natl Acad Sci USA 96(5), 1886-91 (1999)). However, most existing vectors cause gene expression in the nucleus, and therefore the efficiency of nuclear export of the expressed ribozymes is a large hurdle.

### Disclosure of the Invention

An objective of the present invention is to provide paramyxoviral vectors encoding RNAs with catalytic activity, and uses thereof.

Paramyxoviruses have no DNA phase in their transcription and replication, and their gene expression occurs in the cytoplasm. Thus, the present inventors conceived that paramyxoviruses could be vectors potentially suited to ribozyme expression. However, paramyxoviral vectors had not been used to express ribozymes in the past, and it was not known whether active ribozymes could be transcribed and function in cells. Thus, to develop paramyxoviral vectors that could express active ribozymes, the present inventors inserted genes encoding ribozymes into Sendai virus (SeV) vectors, infected the vectors to cells, and evaluated the effect. K-ras, whose mutation frequency is known to be high in colorectal cancer, was chosen as a target. Ras protein is one of the G proteins involved in signal transduction in cells, and substituting valine for the residue at amino acid 12 results in canceration of cells. A number of experiments using ribozymes targeted to regions containing the mutation at amino acid 12 have been previously reported (Funato, T. *et al.,* Cancer Gene Ther 7(3), 495-500 (2000); Tsuchida, T. *et al.,* Cancer Gene Ther 7(3), 373-83 (2000); Tsuchida, T. *et al.,* Biochem Biophys Res Commun 253(2), 368-73 (1998); Zhang, Y.A. *et al.,* Gene Ther 7(23), 2041-50 (2000)). In the present invention, in addition to previously reported K-ras ribozymes, ribozymes with K-ras recognition sites of different length were designed and their effects observed. It was found that K-ras expression in cells could be significantly suppressed by infecting paramyxoviral vectors encoding the ribozymes. In addition, it was discovered that when the length of the ribozyme was 50 nucleotides or more, it was possible to markedly improve the activity of the ribozyme expressed from the vector, as compared with shorter ribozymes. When the length of the ribozyme was 60 nucleotides or more, the activity of the ribozyme introduced with the vector further improved, and when the length was 70 nucleotides or more, the activity was even higher.

Thus, the present invention succeeded in expressing active ribozymes using paramyxoviral vectors for the first time, and demonstrated that the activity of a ribozyme expressed from a paramyxoviral vector could be improved by adjusting the length of ribozyme carried by the vector. Paramyxovirus infection occurs in a broad range of tissues. Accordingly, the use of paramyxoviral vectors encoding ribozymes makes it possible to achieve ribozyme gene therapy directed at tissues and cells to which gene introduction has been problematic using conventional vectors. For example, gene therapy for various cancers can be achieved by inhibiting the expression of oncogenes and the like using the paramyxoviral vectors encoding ribozymes of the present invention. Thus, the paramyxoviral vectors for expressing ribozymes are applicable to various clinical cases.

The present invention relates to paramyxoviral vectors encoding ribozymes and to uses thereof. More specifically, the present invention relates to each of the inventions set forth in the claims. The present invention also relates to inventions comprising a desired combination of one or more (or all) inventions set forth in the claims, in particular, to inventions comprising a desired combination of one or more (or all) inventions set forth in those claims (dependent claims) citing the same independent claim(s) (claim(s) relating to inventions not encompassed by the inventions recited in other claims). The inventions set forth in each independent claim are also intended to include any combinations of the inventions set forth in the dependent claims. Specifically, the present invention includes:
(1) a paramyxoviral vector encoding RNA having a catalytic activity;
(2) the paramyxoviral vector of (1), wherein the length of the RNA is 50 nucleotides or more;
(3) the paramyxoviral vector of (1), wherein the length of the RNA is 60 nucleotides or more;
(4) the paramyxoviral vector of (1) to (3), wherein the RNA is a hammerhead ribozyme;
(5) the paramyxoviral vector of (1) to (4), wherein the paramyxovirus is Sendai virus.

In the present invention, a recombinant virus means a virus produced via a recombinant polynucleotide. A recombinant polynucleotide is a polynucleotide whose binding has been artificially modified (cleaved or linked). Recombinant polynucleotides are not bound in a natural manner. Recombinant polynucleotides can be produced by gene recombination methods known in the art, by combining polynucleotide syntheses, nuclease treatments, ligase treatments, and so on. Recombinant proteins can be produced by expressing recombinant polynucleotides that encode the proteins. Recombinant viruses can be produced by expressing polynucleotides that encode viral genomes constructed by gene manipulations, and then reconstituting the viruses. "Recombinant proteins" comprise proteins produced via recombinant polynucleotides, and artificially synthesized proteins.

In the present invention, a "gene" refers to a genetic substance, a nucleic acid encoding a transcription unit. Genes may be RNAs or DNAs. In this invention, a nucleic acid encoding a protein is referred to as a gene of that protein. Further, a nucleic acid encoding a ribozyme is referred to as a gene of the ribozyme. A gene may be a naturally occurring or artificially designed sequence. Furthermore, in the present invention, "DNA" includes both single-stranded and double-stranded DNAs. Moreover, "encoding a protein or a ribozyme" means that a polynucleotide comprises a nucleic acid or a ribozyme that encodes an amino acid sequence of the protein or the ribozyme in a sense or antisense strand, so that the protein can be expressed under appropriate conditions. The paramyxoviral genes are encoded as antisense sequences in the viral genome.

The paramyxoviral vectors of the present invention operatively encode RNAs with catalytic activity (ribozyme). Specifically, the paramyxoviral vectors of the present invention carry an antisense ribozyme RNA sequence between the 3'-leader sequence and 5'-trailer sequence in the viral genome.

In this invention, a paramyxovirus refers to a virus belonging to Paramyxoviridae, or to derivatives thereof. Paramyxoviruses are a group of viruses with non-segmented negative strand RNA as their genome, and they include Paramyxovirinae (including *Respirovirus* (also referred to as *Paramyxovirus), Rubulavirus,* and *Morbillivirus),* and Pneumovirinae (including *Pneumovirus* and *Metapneumovirus).* Specific examples of *Paramyxovirus* applicable to the present invention are Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3.

Viruses of this invention are preferably those belonging to Paramyxovirinae or derivatives thereof, and more preferably those belonging to the genus *Respirovirus* or derivatives thereof. Examples of viruses of the genus *Respirovirus* applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). The most preferred paramyxovirus in this invention is Sendai virus. These viruses may be derived from natural strains, wild strains, mutant strains, laboratory-passaged strains, artificially constructed strains, or the like.

In this invention, a "vector" is a carrier for introducing a nucleic acid into a cell. Paramyxoviral vectors are carriers derived from paramyxoviruses to introduce nucleic acids into cells. Paramyxoviruses such as SeV described above are excellent gene transfer vectors. Since paramyxoviruses carry out transcription and replication only in the cytoplasm of host cells, and since they don't have a DNA phase, chromosomal integration does not occur. Therefore, they do not give rise to safety problems caused by chromosomal abberations, such as canceration or immortalization. This characteristic of paramyxoviruses contributes a great deal to safety when using a paramyxovirus as a vector. When used for foreign gene expression, SeV showed hardly any nucleotide mutation, even after continuous multiple passaging, indicating the high stability of its genome and the long-term stable expression of inserted foreign genes (Yu, D*. et al.,* Genes Cells 2, 457-466 (1997)). SeV has further qualitative merits, such as flexibility in the size of the genes to be inserted and in the packaging thereof, since it does not have a capsid structure protein. A replicable SeV vector can introduce a foreign gene of at least 4 kb in size, and can simultaneously express two or more genes by adding transcription units. Thus, two or more ribozymes can be expressed from the same vector.

SeV is known to be pathogenic to rodents, causing pneumonia; however, it is not pathogenic to humans. This was supported by a previous report that nasal administration of wild type SeV to non-human primates does not show severe adverse effects (Hurwitz, J. L*. et al.,* Vaccine 15: 533-540, 1997). The two points below, "high infectivity" and "high expression level", should also be noted as advantages. SeV vectors infect cells by binding to sialic acids in the sugar chains of cell membrane proteins or glycolipids. This sialic acid is expressed in almost all cells, giving rise to a broad infection spectrum, *i.e.,* high infectivity. When a replicable SeV replicon-based vector releases viruses, these viruses re-infect neighboring cells, replicating multiple ribonucleoprotein (RNP) copies in the cytoplasm of infected cells, and distributing these into daughter cells in line with cell division, and therefore continuous expression can be expected. Further, SeV vectors can be applied to an extremely wide range of tissues. Furthermore, their characteristic expression mechanism, wherein transcription and replication occurs only in the cytoplasm, has been shown to express inserted genes at very high levels (Moriya, C*. et al.,* FEBS Lett. 425(1) 105-111 (1998); WO00/70070). Furthermore, SeV vectors made non-transmissible by deleting an envelope gene have been successfully recovered (WO00/70070; Li, H.-O*. et al.,* J. Virol. 74(14) 6564-6569 (2000)). Thus, SeV vectors have been improved to further enhance their "safety", while maintaining their "high infectivity" and "high expression levels".

These characteristics of SeV support the effectivity of paramyxoviral vectors including SeV for gene therapy and gene transfer, and the likelihood that SeV will become a promising choice in gene therapy for *in vivo* or *ex vivo* ribozyme expression. By inserting a ribozyme-encoding gene for treatment (or analysis) into a paramyxoviral vector, and administering the vector locally, the ribozyme gene can be locally expressed at high levels, and definite therapeutic effects can be expected, along with reduced side effects. These effects are thought to be stronger for those paramyxoviral vectors, including SeV, that can induce strong transient expression of inserted genes.

Paramyxoviral vectors comprise paramyxovirus genomic RNAs. A genomic RNA refers to an RNA that comprises the function of forming an RNP with a viral protein of a paramyxovirus, such that a gene in the genome is expressed by the protein, and that nucleic acid is replicated to form daughter RNPs. Paramyxoviruses are viruses with a single-strand negative chain RNA in their genome, and such RNAs encode genes as antisense sequences. In general, in the paramyxoviral genome, viral genes are arranged as antisense sequences between the 3'-leader region and the 5'-trailer region. Between the ORFs of respective genes are a transcription ending sequence (E sequence) - intervening sequence (I sequence) - transcription starting sequence (S sequence), such that each gene is transcribed as an individual cistron. Genomic RNAs in a vector of this invention comprise the antisense RNA sequences encoding N (nucleocapsid)-, P (phospho)-, and L (large)-proteins, which are viral proteins essential for the expression of the group of genes encoded by an RNA, and for the autonomous replication of the RNA itself. The RNAs may also encode M (matrix) proteins, essential for virion formation. Further, the RNAs may encode envelope proteins essential for virion infection. Paramyxovirus envelope proteins include F (fusion) protein that causes cell membrane fusion, and HN (hemagglutinin-neuraminidase) protein, essential for viral adhesion to cells. However, HN protein is not required for the infection of certain types of cells (Markwell, M*.*A*. et al.,* Proc. Natl. Acad. Sci. USA 82(4): 978-982 (1985)), and infection is achieved with F protein only. The RNAs may encode envelope proteins other than F protein and/or HN protein.

Paramyxoviral vectors of this invention may be, for example, complexes of paramyxoviral genomic RNAs and viral proteins, that is, ribonucleoproteins (RNPs). RNPs can be introduced into cells, for example, in combination with desired transfection reagents. Specifically, such RNPs are complexes comprising a paramyxoviral genomic RNA, N protein, P protein, and L protein. On introducing an RNP into cells, cistrons encoding the viral proteins are transcribed from the genomic RNA by the action of viral proteins, and, at the same time, the genome itself is replicated to form daughter RNPs. Replication of a genomic RNA can be confirmed by using RT-PCR, Northern blot hybridization, or the like to detect an increase in the copy number of the RNA.

Further, paramyxoviral vectors of this invention are preferably paramyxovirus virions. "Virion" means a microparticle comprising a nucleic acid released from a cell by the action of viral proteins. Paramyxovirus virions comprise structures in which an above-described RNP, comprising genomic RNA and viral proteins, is enclosed in a lipid membrane (referred to as an envelope), derived from the cell membrane. Virions may have infectivity. Infectivity refers to the ability of a paramyxoviral vector to introduce nucleic acids in the vector into cells to which the virion has adhered, since they retain cell adhesion and membrane-fusion abilities. Paramyxoviral vectors of this invention may be replicable or replication-deficient vectors. "Replicable" means that when a viral vector is introduced into a host cell, the virus can replicate itself within the cell to produce infectious virions.

For example, each gene in each virus belonging to Paramyxovirinae is generally described as below. In general, N gene is also described as "NP".

| | | | | | | | |
|---|---|---|---|---|---|---|---|
| *Respirovirus* | NP | P/C/V | M | F | HN | - | L |
| *Rubulavirus* | NP | P/V | M | F | HN | (SH) | L |
| *Morbillivirus* | NP | P/C/V | M | F | H | - | L |

For example, the database accession numbers for the nucleotide sequences of each of the Sendai virus genes are: M29343, M30202, M30203, M30204, M51331, M55565, M69046, and X17218 for N gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene.

The ORFs of these viral proteins are arranged as antisense sequences in the genomic RNAs, via the above-described E-I-S sequence. The ORF closest to the 3'-end of the genomic RNAs only requires an S sequence between the 3'-leader region and the ORF, and does not require an E or I sequence. Further, the ORF closest to the 5'-end of the genomic RNA only requires an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. In wild type paramyxoviruses, a typical RNA genome comprises a 3'-leader region, six ORFs encoding the N, P, M, F, HN, and L proteins in the antisense and in this order, and a 5'-trailer region on the other end. In the genomic RNAs of this invention, as for the wild type viruses, it is preferable that ORFs encoding the N, P, M, F, HN, and L proteins are arranged in this order, after the 3'-leader region, and before the 5'-trailer region; however, the gene arrangement is not limited to this. Certain types of paramyxovirus do not comprise all six of these viral genes, but even in such cases, it is preferable to arrange each gene as in the wild type, as described above. In general, vectors maintaining the N, P, and L genes can autonomously express genes from the RNA genome in cells, replicating the genomic RNA. Furthermore, by the action of genes such as the F and HN genes, which encode envelope proteins, and the M gene, infectious virions are formed and released to the outside of cells. Thus, such vectors become replicable viral vectors. A gene encoding a ribozyme may be inserted into a protein-noncoding region between the 3'-leader sequence and 5'-trailer sequence in this genome, as described below.

Further, a paramyxoviral vector of this invention may be deficient in any of the wild type paramyxoviral genes. For example, a paramyxoviral vector that is inactivated in or deficient in the M, F, or HN gene, or any combinations thereof, can be preferably used as a paramyxoviral vector of this invention. Such viral vectors can be reconstituted, for example, by externally supplying the products of the deficient genes. The viral vectors thus prepared adhere to host cells to cause cell fusion, as for wild type viruses, but they cannot form daughter virions that comprise the same infectivity as the original vector, because the vector genome introduced into cells is deficient in a viral gene. Therefore, such vectors are useful as safe viral vectors that can only introduce genes once. Examples of genes that the genome may be defective in are the F gene, HN gene, M gene, and any combinations of two or three of them. For example, viral vectors can be reconstituted by transfecting host cells with a plasmid expressing a recombinant paramyxoviral vector genome defective in the F gene, along with an F protein expression vector and expression vectors for the NP, P, and L proteins (WO00/70055, WO00/70070, WO03/025570; Li, H.-O*. et al.,* J. Virol. 74(14) 6564-6569 (2000)). Viruses can also be produced by, for example, using host cells that have incorporated the F gene into their chromosomes. When supplying these proteins externally, their amino acid sequences do not need to be the same as the viral sequences, and a mutant or homologous gene from another virus may be used as a substitute, as long as their activity in nucleic acid introduction is the same as, or greater than, that of the natural type.

Further, vectors that comprise an envelope protein other than that of the virus from which the vector genome was derived, may be prepared as viral vectors of this invention. For example, when reconstituting a virus, a viral vector comprising a desired envelope protein can be generated by expressing an envelope protein other than the original envelope protein encoded by the basic viral genome. Such proteins are not particularly limited, and include the envelope proteins of other viruses, for example, the G protein of vesicular stomatitis virus (VSV-G). The viral vectors of this invention include pseudotype viral vectors comprising envelope proteins, such as VSV-G, derived from viruses other than the virus from which the genome was derived. By designing the viral vectors such that these envelope proteins are not encoded in RNA genomes, the proteins will never be expressed after virion infection of the cells.

Furthermore, a viral vector of this invention may be, for example, a vector with, on the envelope surface, a protein that can attach to a specific cell, such as an adhesion factor, ligand, receptor, antibody, or fragment thereof; or a vector comprising a chimeric protein with such a protein in the extracellular domain, and a polypeptide derived from the virus envelope in the intracellular domain. Thus, vectors that target specific tissues can also be produced. Such proteins may be encoded by the viral genome, or supplied by expressing genes other than the viral genome at the time of viral vector reconstitution (for example, other expression vectors or genes existing on host chromosomes).

Further, in the vectors of this invention, any viral gene comprised in the vector may be modified from the wild type gene in order to reduce the immunogenicity caused by viral proteins, or to enhance RNA transcriptional or replicational efficiency, for example. Specifically, for example, in a paramyxoviral vector, modifying at least one of the N, P, and L genes, which are replication factors, is considered to enhance transcriptional or replicational function. Further, HN protein, which is an envelope protein, comprises both hemagglutinin activity and neuraminidase activity; however, it is possible, for example, to improve viral stability in the blood if the former activity can be attenuated, and infectivity can be controlled if the latter activity is modified. Further, it is also possible to control membrane fusion ability by modifying F protein. For example, the epitopes of the F protein or HN protein, which can be cell surface antigenic molecules, can be analyzed, and using this, viral vectors with reduced antigenicity to these proteins can be prepared.

Furthermore, vectors of this invention may be deficient in accessory genes. For example, by knocking out the V gene, one of the SeV accessory genes, the pathogenicity of SeV toward hosts such as mice is remarkably reduced, without hindering gene expression and replication in cultured cells (Kato, A*. et al.,* 1997, J. Virol. 71: 7266-7272; Kato, A. *et al.,* 1997, EMBO J. 16: 578-587; Curran, J. *et al.,* WO01/04272, EP1067179). Such attenuated vectors are particularly useful as nontoxic viral vectors for *in vivo* or *ex vivo* gene transfer.

The vectors of the present invention contain RNAs encoding ribozymes in the genome of the above-described paramyxoviral vectors. Herein, a "ribozyme" refers to an RNA with catalytic activity (T. R. Cech *et al.,* Cell, 27:487-496 (1981); Koizumi M. and Otsuka E., Tanpakushitsu Kakusan Koso (Protein, Nucleic acid, Enzyme), 35:2191 (1990)). The catalytic activity is not limited and can include, for example, cleavage and ligation of RNA or DNA strands, template-dependent RNA replication, RNA aminoacylation, amino acid transfer from tRNA to a 5' hydroxyl group, autophosphorylation, nucleotide synthesis, and the formation of peptide bonds. A particularly important activity is RNA-cleaving activity. In the present invention, the vector can carry any ribozymes, and such ribozymes include, for example, hammerhead ribozymes, hairpin ribozymes, Delta hepatitis virus ribozymes, RNA subunits of ribonuclease P, group I and II introns, and altered forms thereof. Herein, an "alteration" refers to the substitution, deletion, or addition of one or more nucleotides in a nucleic acid sequence. There are conventional methods for obtaining altered ribozymes with higher activities, in which natural ribozymes are altered by *in-vitro* evolution systems (Joyce, Selective Amplification Techniques for Optimization of Ribozyme Function. in "Antisense RNA and DNA", pp. 353-372 (1992) Wiley-Liss Inc.). The ribozymes may function as dimers.

In the present invention, hammerhead ribozyme and hairpin ribozyme are particularly preferred ribozymes to be carried by the vectors. Natural hammerhead ribozymes have been isolated from viroids and the like (J. M. Buzayan *et al.,* Nature, 323: 349-353 (1986); GA. Prody *et al.,* Science, 231:1577-1580 (1986)). The hammerhead ribozymes have a hammerhead structure comprising three loops and three helices and originally act in cis, but can act in trans when the RNA portion with catalytic activity is separated from the target RNA. Such a ribozyme might comprise, for example, one loop and one helix, and form a pseudo-loop with the target sequence (Turner, P.C., The Biochemistry of the Hammerhead Ribozyme. In: Scanlon, KJ., and Kashani-Sabet, M. ed. "Ribozymes in the Gene Tarapy of Cancer (Medical Intelligence UNIT4)", R. G Landes Company, 3-14 (1998)). The structure of hammerhead ribozymes has been well analyzed. The ribozyme of tobacco ringspot virus specifically cleaves the 3' end of the nucleotide sequence NUH (N is A, G, C, or U; H is A, C, or U) (M. Koizumi *et al.,* FEBS Lett. 228:225 (1988)). Thus, a ribozyme can be created to specifically cleave a site comprising UC, UU, or UA in a desired target RNA sequence (M. Koizumi *et al.,* FEBS Lett. 239:285 (1988); Koizumi M. and Otsuka E., Tanpakushitsu Kakusan Koso (Protein, Nucleic acid, Enzyme), 35: 2191 (1990); M. Koizumi *et al.,* Nucleic Acids Res. 17:7059 (1989)).

In addition, hairpin ribozymes can also be used to achieve the purpose of the present invention. Hairpin ribozymes exist, for example, in the minus strand of satellite RNA of tobacco ringspot virus (J. M. Buzayan, Nature 323:349 (1986)). This ribozyme can also be designed to achieve target-specific RNA cleavage (Y. Kikuchi and N. Sasaki, Nucleic Acids Res. 19:6751 (1992); Y. Kikuchi, Kagaku To Seibutsu (Chemistry amd Biology) 30:112 (1992)).

In the present invention, the length of a ribozyme carried by a vector is preferably 50 nucleotides or more, more preferably 55 nucleotides or more, even more preferably 60 nucleotides or more, still more preferably 65 nucleotides or more, yet more preferably 70 nucleotides or more, and yet still more preferably 75 nucleotides or more. The "length of a ribozyme" refers to the number of nucleotides of a ribozyme RNA molecule transcribed from the vector. If the ribozyme is too short, its length can be increased preferably by increasing the size of the target recognition sequence. For example, one of advantages of the present invention is that even if its length is 45 nucleotides or less, or 40 nucleotides or less, an active ribozyme can be expressed from paramyxoviral vectors as a highly active ribozyme by adjusting the length of the target recognition sequence.

In general, a ribozyme with RNA-cleaving activity contains sequences essential for catalytic activity and sequences required for target RNA recognition. Sequences required for the catalytic activity of hammerhead ribozymes include, for example, 5'-¹C²U³G⁴A⁵N⁶G⁷A⁸N⁹N¹⁰N¹¹N¹²N¹³N¹⁴N¹⁵N¹⁶N¹⁷N¹⁸N¹⁹N²⁰G²¹A²²A²³A²⁴N-3' (SEQ ID NO: 38) (where N represents G, A, U, or C; 5'-⁸N⁹N¹⁰N¹¹N-3' and 5'-¹⁶N¹⁷N¹⁸N¹⁹N-3' are designed to be complementary to each other to allow base pairing), but are not limited thereto. The four nucleotides ¹²N¹³N¹⁴N¹⁵N preferably form a loop. Instead of four nucleotides, the number of nucleotides may range from about 2 to 7 nucleotides (i.e. N₂₋₇), for example, 3 to 5 nucleotides (i.e. N₃₋₅),. ²³A²⁴N overlaps the target recognition sequence. ²⁴N is a nucleotide complementary to the N of the target site NUH described above. More specific sequences are shown in the Examples. The target recognition sequence is attached to both ends. Specifically, the target recognition sequence refers to a portion comprising a sequence complementary to the target RNA. In the present invention, the length of the target recognition site of a ribozyme is preferably 20 nucleotides or more, more preferably 25 nucleotides or more, even more preferably 30 nucleotides or more, still more preferably 35 nucleotides or more, yet more preferably 40 nucleotides or more, and yet still more preferably 45 nucleotides or more. When the target recognition site is split into discrete sequence stretches, the length corresponds to the total sum of the respective nucleotides. For example, the target recognition site for hammerhead ribozymes is generally at both ends of the ribozyme RNA, and therefore the length corresponds to the total sum of nucleotides complementary to the target RNA at these two ends. It is preferable to add to each end a sequence complementary to the target RNA, which comprises at least consecutive 7 nucleotides, preferably consecutive 8 nucleotides or more, more preferably consecutive 9 nucleotides or more, even more preferably consecutive 10 nucleotides or more, still more preferably consecutive 12 nucleotide or more, and yet more preferably consecutive 15 nucleotides or more.

A nucleic acid encoding a ribozyme can be inserted at any desired position in a protein-noncoding region between the 3'-leader sequence and 5'-trailer sequence in the genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region. Further, in genomes deficient in the F or HN gene or the like, nucleic acids encoding ribozymes can be inserted into those deficient regions. When introducing a foreign gene into a paramyxovirus, it is desirable to insert the gene such that the chain length of the polynucleotide to be inserted into the genome will be a multiple of six (Journal of Virology, Vol. 67, No. 8, 4822-4830, 1993). An E-I-S sequence should be arranged between the inserted foreign gene and the viral protein ORF. Two or more nucleic acids encoding ribozymes can be inserted in tandem via E-I-S sequences. Alternatively, a desired gene may be inserted though an IRES (internal ribosome entry site).

Expression levels of a ribozyme carried in a vector can be controlled using the type of transcriptional initiation sequence added upstream (to the 3'-side of the minus strand (the negative strand)) of the gene (WO01/18223). The expression levels can also be controlled of the position at which the foreign gene encoding a ribozyme is inserted in the genome: the nearer to the 3'-end of the minus strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high expression level of the ribozyme is thought to be advantageous, it is preferable to link a gene encoding a ribozyme to a highly efficient transcriptional initiation sequence, and to insert it near the 3'-end of the minus strand genome. Specifically, a foreign gene is inserted between the 3'-leader region and the viral protein ORF closest to the 3'-end. Alternatively, a foreign gene may be inserted between the ORFs of the viral protein gene closest to the 3'-end and the second closest viral gene, or between the second closest and the third closest viral protein genes to the 3'-end. In typical paramyxoviruses, the viral protein gene closest to the 3'-end of the genome is the N gene, the second closest gene is the P gene, and the third closest gene is the M gene. Alternatively, when a high level of expression of the introduced gene is undesirable, the gene expression level from the viral vector can be suppressed to obtain an appropriate effect, for example, by inserting the foreign gene at a site in the vector as close as possible to the 5'-side of the minus strand, or by selecting an inefficient transcriptional initiation sequence.

The RNA encoding ribozyme is arranged and flanked by an S sequence and E sequence in the paramyxoviral genome. When the ribozyme is transcribed from the vector, transcription is initiated from the S sequence and terminated at the E sequence. The ribozyme transcript thus contains an extra sequence at each end of the sequence originally required for ribozyme activity. These extra sequences, which are added by inserting a nucleic acid that encodes a ribozyme into a paramyxoviral vector, are herein called "spacers". Specifically, the spacers are sequences attached to both ends of a ribozyme transcribed from a paramyxoviral vector and are not involved in the target recognition and catalytic activity of the ribozyme (however, this does not include the poly(A) tail attached to the 3' end). Spacers include portions of the S sequence and E sequence, and linker sequences used to insert ribozymes into the vectors. As described in the Examples, even when spacers are attached to the ends of the ribozyme, the ribozyme retains its activity. The total length of spacers at the ends of the ribozyme may be 10 nucleotides or more; when the ribozyme is longer, the length may be 20 nucleotides or more; when the ribozyme is even longer, the length may 25 nucleotides or more; when the ribozyme is much longer, the length may 30 nucleotides or more; and when the ribozyme is still longer, the length may 35 nucleotides or more (the poly(A) is not included in the calculation of length). However, the total length of spacers at the two ends of the ribozyme is 100 nucleotides or less, preferably 80 nucleotides or less, more preferably 70 nucleotides or less, even more preferably 60 nucleotides or less, still more preferably 50 nucleotides or less. The lengths of the spacers at each end are preferably 50 nucleotides each or less.

For example, a desired S sequence of paramyxovirus may be used as the S sequence to be attached when a nucleic acid encoding a ribozyme is inserted into the genome. The sequence 3'-UCCCWVUUWC-5' (W= A or C; V= A, C, or G)(SEQ ID NO: 1) can preferably be used when the vector is a Sendai virus vector. Particularly preferred sequences are 3'-UCCCAGUUUC-5' (SEQ ID NO: 2), 3'-UCCCACUUAC-5' (SEQ ID NO: 3), and 3'-UCCCACUUC-5' (SEQ ID NO: 4). When shown as DNA sequences encoding the plus strand these sequences are 5'-AGGGTCAAAG-3' (SEQ ID NO: 5), 5'-AGGGTGAATG-3' (SEQ ID NO: 6), and 5'-AGGGTGAAAG-3' (SEQ ID NO: 7) . A preferred E sequence of Sendai viral vector is, for example, 3'-AUUCUUUUU-5' (SEQ ID NO: 8) (5'-TAAGAAAAA-3' (SEQ ID NO: 9) for a DNA encoding the plus strand). An I sequence may comprise, for example, any three nucleotides, specifically 3'-GAA-5' (5'-CTT 3' in the plus strand DNA).

Ribozymes can be transcribed from the vectors of the present invention by infecting the vectors to cells. For example, when the vectors encode ribozymes with the activity of cleaving transcripts of genes expressed in cells, infecting the vectors to cells can suppress the expression of target genes. The vectors of the present invention encoding ribozymes with the activity of cleaving transcripts of the target genes have the ability to decrease expression levels of target genes to preferably 70% or less, more preferably 75% or less, and still more preferably 60% or less when they infect cells expressing the target genes at MOI=10, as compared with a control vector at the same MOI that does not encode a ribozyme.

The vectors of the present invention can be used as therapeutic viral vectors that can be administered *in vivo.* The vectors of the present invention are safe since they are not integrated into host chromosomes. The vectors are applicable to the treatment of various diseases or disorders, because they typically allow expression of ribozymes for several days to several weeks or longer. The local administration of a vector of the present invention for therapeutic purposes may result in *in-vivo* local overexpression of ribozymes (clinical applications). In particular, ribozymes are a potentially useful tool in gene therapy for cancers (H. Kijima *et al.,* Pharmac. Ther., 68:247-267 (1995); A. Irie *et al.,* Int. J. Urol., 4:329-337 (1997)). In addition to application to the treatment of cancers, various applications for ribozymes can be envisaged. For example, gene therapy using the vectors of the present invention that encode ribozymes can be used to prevent and treat infectious diseases, including AIDS (Rossi, J.J., Curr. Opin. Mol. Ther., 1(3):316-22 (1999); Lewin, A.S. and Hauswirth, W.W., Trends Mol. Med., 7(5):221-8 (2001); Gaughan, D.J. et al., Biochim. Biophys. Acta, 1445(1):1-20 (1999)).

To prepare a vector of the present invention, a cDNA encoding a genomic RNA of a paramyxovirus of this invention is transcribed in mammalian cells, in the presence of viral proteins (i.e., N, P, and L proteins) essential for reconstitution of an RNP, which comprises a genomic RNA of a paramyxovirus. Viral RNP can be reconstituted by producing either the minus strand genome (also called the negative strand, the same antisense strand as the viral genome) or the plus strand (also called the positive strand, the sense strand encoding the viral proteins). Production of the plus strand is preferable for increased efficiency of vector reconstitution. The RNA terminals preferably reflect the terminals of the 3'-leader sequence and 5'-trailer sequence as accurately as possible, as in the natural viral genome. To accurately regulate the 5'-end of the transcript, for example, the RNA polymerase may be expressed within a cell using the recognition sequence of T7 RNA polymerase as a transcription initiation site. Furthermore, the 3'-end of the transcript is cleaved accurately by the autocleavage (Hasan, M. K. *et al.,* J. Gen. Virol. 78: 2813-2820 (1997); Kato, A*. et al.,* EMBO J. 16: 578-587 (1997); and Yu, D. *et al.,* Genes Cells 2: 457-466 (1997)).

For example, a recombinant Sendai virus vector carrying a ribozyme can be constructed as follows, according to descriptions in: Hasan, M. K*. et al.,* J. Gen. Virol. 78: 2813-2820 (1997); Kato, A*. et al.,* EMBO J. 16: 578-587 (1997); Yu, D*. et al.,* Genes Cells 2: 457-466 (1997); or the like.

First, a DNA that encodes a ribosome is synthesized such that there are restriction sites for cloning at both ends. The synthetic DNA should contain an E-I-S sequence such that the E-I-S sequence is placed between the inserted ribozyme and ORFs of the flanking viral genes in the viral genome. The synthetic DNA is designed such that its size (the number of nucleotides) is a multiple of 6 when inserted into the vector (the so-called "rule of six"; Kolakofski, D. et al., J. Virol. 72:891-899 (1998); Calain, P. and Roux, L., J. Virol. 67:4822-4830 (1993); Calain, P. and Roux, L., J. Virol. 67: 4822-4830 (1993)). As the E-I-S sequence, for example, S, I, and E sequences of the minus strand of Sendai virus, preferably the sequences 5'-CTTTCACCCT 3' (SEQ ID NO: 10), 5'-AAG-3', and 5'-TTTTTCTTACTACGG-3' (SEQ ID NO: 11) respectively, are placed at the 3' end of the oligo DNA insert fragment. This fragment is inserted into a cDNA encoding the viral genome. For example, the fragment is inserted between the ORFs encoding the viral proteins in the genome and the upstream S sequence so that E-I-S sequences are placed one by one between the ORFs of the viral protein genes on either side of the ribozyme.

For example, a cDNA for a recombinant Sendai virus genome can be constructed by methods described in previous reports (Yu, D. *et al.,* Genes Cells 2: 457-466 (1997); Hasan, M. K*. et al.,* J. Gen. Virol. 78: 2813-2820 (1997)). For example, a double-stranded DNA is synthesized to have an E-I-S sequence attached to the 3'end of the sense strand of the ribozyme. The DNA is inserted immediately 3' of a desired S sequence in a cDNA encoding the sense strand of the genome. For example, a restriction site is first placed between a sequence encoding a desired viral protein gene and an S sequence involved in the transcription of the gene in a cDNA encoding the plus strand genome. A DNA encoding a ribozyme-E-I-S sequence can then be inserted into the restriction site (Tokusumi, T. *et al.* Virus Res 86(1-2), 33-8 (2002)). Specifically, for example, high efficiency virus production can be achieved by inserting the sequence between an S sequence flanked by P and M genes and the ORF for M gene.

A vector of this invention can be reconstituted by transcribing a DNA encoding a genomic RNA of a recombinant paramyxovirus thus prepared, in cells in the presence of the above-described viral proteins (L, P, and N). The present invention provides DNAs encoding the viral genomic RNAs of the vectors of this invention, for manufacturing the vectors of this invention. This invention also relates to the use of DNAs encoding the genomic RNAs of the vectors, for their application to the manufacture of the vectors of this invention. The recombinant viruses can be reconstituted by methods known in the art (WO97/16539; WO97/16538; Durbin, A. P. *et al.,* Virology 235: 323-332 (1997); Whelan, S. P. *et al.,* Proc. Natl. Acad. Sci. USA 92: 8388-8392 (1995); Schnell. M. J. *et al.,* EMBO J. 13: 4195-4203 (1994); Radecke, F. *et al.,* EMBO J. 14: 5773-5784 (1995); Lawson, N. D*. et al.,* Proc. Natl. Acad. Sci. USA 92: 4477-4481; Garcin, D*. et al.,* EMBO J. 14: 6087-6094 (1995); Kato, A*. et al.,* Genes Cells 1: 569-579 (1996); Baron, M. D. and Barrett, T., J. Virol. 71: 1265-1271 (1997); Bridgen, A. and Elliott, R. M., Proc. Natl. Acad. Sci. USA 93: 15400-15404 (1996)). With these methods, minus strand RNA viruses including parainfluenza virus, vesicular stomatitis virus, rabies virus, measles virus, rinderpest virus, and Sendai virus can be reconstituted from DNA. The vectors of this invention can be reconstituted according to these methods. When a viral vector DNA is made F gene, HN gene, and/or M gene deficient, such DNAs do not form infectious virions as is. However, by separately introducing host cells with these lacking genes, and/or genes encoding the envelope proteins of other viruses, and then expressing these genes therein, it is possible to form infectious virions.

Specifically, the viruses can be prepared by the steps of: (a) transcribing cDNAs encoding paramyxovirus genomic RNAs (minus strand RNAs), or complementary strands thereof (plus strands), in cells expressing N, P, and L proteins; and (b) harvesting complexes that comprise the genomic RNAs from these cells, or from culture supernatants thereof. For transcription, a DNA encoding a genomic RNA is linked downstream of an appropriate promoter. The genomic RNA thus transcribed is replicated in the presence of N, L, and P proteins to form an RNP complex. Then, in the presence of M, HN, and F proteins, virions enclosed in an envelope are formed. For example, a DNA encoding a genomic RNA can be linked downstream of a T7 promoter, and transcribed to RNA by T7 RNA polymerase. Any desired promoter, other than those comprising a T7 polymerase recognition sequence, can be used as a promoter. Alternatively, RNA transcribed *in vitro* may be transfected into cells.

Enzymes essential for the initial transcription of genomic RNA from DNA, such as T7 RNA polymerase, can be supplied by transducing the plasmid or viral vectors that express them, or, for example, by incorporating a gene thereof into a chromosome of the cell so as to enable induction of their expression, and then inducing expression at the time of viral reconstitution. Further, genomic RNA and viral proteins essential for vector reconstitution are supplied, for example, by transducing the plasmids that express them. In supplying these viral proteins, helper viruses such as wild type or certain types of mutant paramyxovirus can be used, but this may induce contamination of these viruses, and hence is not preferred.

Methods for transducing DNAs expressing the genomic RNAs into cells include, for example, (i) methods for making DNA precipitates which target cells can internalize; (ii) methods for making complexes comprising DNAs suitable for internalization by target cells, and comprising positive charge characteristics with low cytotoxicity; and (iii) methods for using electric pulses to instantaneously bore pores in the target cell membrane, of sufficient size for DNA molecules to pass through.

For (ii), various transfection reagents can be used. For example, DOTMA (Roche), Superfect (QIAGEN #301305), DOTAP, DOPE, DOSPER (Roche #1811169), and such can be cited. As (i), for example, transfection methods using calcium phosphate can be cited, and although DNAs transferred into cells by this method are internalized by phagosomes, a sufficient amount of DNA is known to enter the nucleus (Graham, F. L. and Van Der Eb, J., Virology 52: 456 (1973); Wigler, M. and Silverstein, S., Cell 11: 223 (1977)). Chen and Okayama investigated the optimization of transfer techniques, reporting that (1) incubation conditions for cells and coprecipitates are 2 to 4% CO₂ 35°C, and 15 to 24 hours, (2) the activity of circular DNA is higher than linear DNA, and (3) optimal precipitation is obtained when the DNA concentration in the precipitate mixture is 20 to 30 µg/ml (Chen, C. and Okayama, H., Mol. Cell. Biol. 7: 2745 (1987)). The methods of (ii) are suitable for transient transfections. Methods for performing transfection by preparing a DEAE-dextran (Sigma #D-9885 M.W. 5x 10⁵) mixture with a desired DNA concentration ratio have been known for a while. Since most complexes are decomposed in endosomes, chloroquine may also be added to enhance the effect (Calos, M. P., Proc. Natl. Acad. Sci. USA 80: 3015 (1983)). The methods of (iii) are referred to as electroporation methods, and are in more general use than methods (i) and (ii) because they are not cell selective. The efficiency of these methods is supposed to be good under optimal conditions for: the duration of pulse electric current, shape of the pulse, potency of electric field (gap between electrodes, voltage), conductivity of buffer, DNA concentration, and cell density.

Of the above three categories, the methods of (ii) are simple to operate and can examine many samples using a large amount of cells, and thus transfection reagents are suitable for the transduction into cells of DNA for vector reconstitution. Preferably, Superfect Transfection Reagent (QIAGEN, Cat No. 301305), or DOSPER Liposomal Transfection Reagent (Roche, Cat No. 1811169) is used, but transfection reagents are not limited to these.

Specifically, virus reconstitution from cDNA can be carried out, for example, as follows:

In a plastic plate of about 24- to 6-wells, or a 100-mm Petri dish or the like, LLC-MK2 cells derived from monkey kidney are cultured till near 100% confluent, using minimum essential medium (MEM) comprising 10% fetal calf serum (FCS) and antibiotics (100 units/ml penicillin G and 100 µg/ml streptomycin), and infected with, for example, 2 PFU/cell of the recombinant vaccinia virus vTF7-3, which expresses T7 RNA polymerase and has been inactivated by 20-minutes of UV irradiation in the presence of 1 µg/ml psoralen (Fuerst, T. R. *et al.,* Proc. Natl. Acad. Sci. USA 83: 8122-8126 (1986); Kato, A*. et al.,* Genes Cells 1: 569-579 (1996)). The amount of psoralen added and the UV irradiation time can be appropriately adjusted. One hour after infection, 2 to 60 µg, and more preferably 3 to 20 µg, of DNA encoding the genomic RNA of a recombinant Sendai virus is transfected along with the plasmids expressing trans-acting viral proteins essential for viral RNP production (0.5 to 24 µg of pGEM-N, 0.25 to 12 µg of pGEM-P, and 0.5 to 24 µg of pGEM-L) (Kato, A*. et al.,* Genes Cells 1: 569-579 (1996)), using the lipofection method or such with Superfect (QIAGEN). The ratio of the amounts of expression vectors encoding the N, P, and L proteins is preferably 2:1:2; and the plasmid amounts are appropriately adjusted, for example, in the range of 1 to 4 µg of pGEM-N, 0.5 to 2 µg of pGEM-P, and 1 to 4 µg of pGEM-L.

The transfected cells are cultured, as occasion may demand, in serum-free MEM comprising 100 µg/ml of rifampicin (Sigma) and cytosine arabinoside (AraC), more preferably only 40 µg/ml of cytosine arabinoside (AraC) (Sigma). Optimal drug concentrations are set so as to minimize cytotoxicity due to the vaccinia virus, and to maximize virus recovery rate (Kato, A*. et al.,* Genes Cells 1: 569-579 (1996)). After culturing for about 48 to 72 hours after transfection, cells are harvested, and then disintegrated by repeating freeze-thawing three times. The disintegrated materials comprising RNP are re-infected to LLC-MK2 cells, and the cells are cultured. Alternatively, the culture supernatant is recovered, added to a culture solution of LLC-MK2 cells to infect them, and then cultured. Transfection can be conducted by, for example, forming a complex with lipofectamine, polycationic liposome, or the like, and transducing the complex into cells. Specifically, various transfection reagents can be used. For example, DOTMA (Roche), Superfect (QIAGEN #301305), DOTAP, DOPE, and DOSPER (Roche #1811169) may be cited. In order to prevent decomposition in the endosome, chloroquine may also be added (Calos, M. P., Proc. Natl. Acad. Sci. USA 80: 3015 (1983)). In cells transduced with RNP, viral gene expression from RNP and RNP replication progress, and the vector is amplified. By diluting appropriately the viral solution (the culture supernatant) thus obtained, and then repeating the amplification, the vaccinia virus vTF7-3 can be completely eliminated. Amplification is repeated, for example, three or more times. Vectors thus obtained can be stored at -80°C. In order to reconstitute a viral vector without replication ability, which is defective in a gene encoding an envelope protein, LLC-MK2 cells expressing the envelope protein may be used for transfection, or a plasmid expressing the envelope protein may be cotransfected. Alternatively, an envelove gene defective type viral vector can be amplified by culturing the transfected cells overlaid with LLK-MK2 cells expressing the envelope protein (see WO00/70055 and WO00/70070).

Titers of viruses thus recovered can be determined, for example, by measuring CIU (Cell Infectious Unit) or hemagglutination activity (HA) (WO00/70070; Kato, A*. et al.,* Genes Cells 1: 569-579 (1996); Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306 (1999)). Titers of vectors carrying GFP (green fluorescent protein) marker genes and the like can be quantified by directly counting infected cells, using the marker as an indicator (for example, as GFP-CIU). Titers thus measured can be handled in the same way as CIU (WO00/70070).

As long as a viral vector can be reconstituted, the host cells used in the reconstitution are not particularly limited. For example, in the reconstitution of Sendai viral vectors and such, cultured cells such as LLC-MK2 cells, CV-1 cells derived from monkey kidney, and BHK cells derived from hamster kidney, and cells derived from humans can be used. By expressing suitable envelope proteins in these cells, infectious virions having envelopes can also be obtained. Further, to obtain a large quantity of a Sendai viral vector, a viral vector obtained from an above-described host can be infected to embrionated hen eggs, to amplify the vector. Methods for manufacturing viral vectors using hen eggs have already been developed (Nakanishi, *et al.,* ed., "State-of-the-Art Technology Protocol in Neuroscience Research III, Molecular Neuron Physiology", Koseisha, Osaka, pp. 153-172 (1993)). Specifically, for example, a fertilized egg is placed in an incubator, and cultured for nine to twelve days at 37 to 38°C to grow an embryo. After the viral vector is inoculated into the allantoic cavity, the egg is cultured for several days (for example, three days) to proliferate the viral vector. Conditions such as the period of culture may vary depending upon the recombinant Sendai virus being used. Then, allantoic fluids comprising the vector are recovered. Separation and purification of a Sendai viral vector from allantoic fluids can be performed according to a usual method (Tashiro, M., "Virus Experiment Protocol", Nagai, Ishihama, ed., Medical View Co., Ltd., pp. 68-73 (1995)).

For example, the construction and preparation of Sendai virus vectors defective in F protein can be performed as described below (see WO00/70055 and WO00/70070):

### <1> Construction of a genomic cDNA of an F defective Sendai virus, and a plasmid expressing F gene

A full-length genomic cDNA of Sendai virus (SeV), the cDNA of pSeV18⁺ b(+) (Hasan, M. K. *et al.,* J. General Virology 78: 2813-2820 (1997)) ("pSeV18⁺ b(+)" is also referred to as "pSeV18⁺"), is digested with *Sph*I/*Kpn*I to recover a fragment (14673 bp), which is cloned into pUC18 to prepare plasmid pUC18/KS. Construction of an F defective site is performed on this pUC18/KS. An F gene deficiency is created by a combination of PCR-ligation methods, and, as a result, the F gene ORF (ATG-TGA = 1698 bp) is removed. Then, for example, atgcatgccggcagatga (SEQ ID NO: 12) is ligated to construct an F defective type SeV genomic cDNA (pSeV18⁺/ΔF). A PCR product formed in PCR by using the pair of primers [forward: 5'-gttgagtactgcaagagc/SEQ ID NO: 13, reverse: 5'-tttgccggcatgcatgtttcccaaggggagagttttgcaacc/SEQ ID NO: 14] is connected upstream of F, and a PCR product formed using the pair of primers [forward: 5'-atgcatgccggcagatga/SEQ ID NO: 15, reverse: 5'-tgggtgaatgagagaatcagc/SEQ ID NO: 16] is connected downstream of F gene at EcoT22I. The plasmid thus obtained is digested with *Sac*I and *Sal*I to recover a 4931 bp fragment of the region comprising the F defective site, which is cloned into pUC18 to form pUC18/dFSS. This pUC18/dFSS is digested with *Dra*III, the fragment is recovered, replaced with the *Dra*III fragment of the region comprising the F gene of pSeV18⁺, and ligated to obtain the plasmid pSeV18⁺/ΔF. A foreign gene is inserted, for example, in to the *Nsi*I and *Ngo*MIV restriction enzyme sites in the F defective site of pUC18/dFSS.

### <2> Preparation of helper cells that induce SeV-F protein expression

To construct an expression plasmid of the Cre/loxP induction type that expresses the Sendai virus F gene (SeV-F), the SeV-F gene is amplified by PCR, and inserted to the unique *Swa*I site of the plasmid pCALNdlw (Arai, T. *et al.,* J. Virology 72, p1115-1121 (1998)), which is designed to enable the inducible expression of a gene product by Cre DNA recombinase, thus constructing the plasmid pCALNdLw/F.

To recover infectious virions from the F defective genome, a helper cell line expressing SeV-F protein is established. The monkey kidney-derived LLC-MK2 cell line, which is commonly used for SeV proliferation, can be used as the cells, for example. LLC-MK2 cells are cultured in MEM supplemented with 10% heat-treated inactivated fetal bovine serum (FBS), penicillin G sodium (50 units/ml), and streptomycin (50 µg/ml) at 37°C in 5% CO₂. Since the SeV-F gene product is cytotoxic, the above-described plasmid pCALNdLw/F, which was designed to enable inducible expression of the F gene product with Cre DNA recombinase, is transfected to LLC-MK2 cells for gene transduction by the calcium phosphate method (using a mammalian transfection kit (Stratagene)), according to protocols well known in the art.

The plasmid pCALNdLw/F (10 µg) is transduced into LLC-MK2 cells grown to 40% confluency using a 10-cm plate, and the cells are then cultured in MEM (10 ml) comprising 10% FBS, in a 5% CO₂ incubator at 37°C for 24 hours. After 24 hours, the cells are detached and suspended in the medium (10 ml). The suspension is then seeded onto five 10-cm dishes, 5 ml to one dish, 2 ml each to two dishes, and 0.2 ml each to two dishes, and cultured in MEM (10 ml) comprising G418 (GIBCO-BRL) (1200 µg/ml) and 10% FBS. The cells were cultured for 14 days, exchanging the medium every two days, to select cell lines stably transduced with the gene. The cells grown from the above medium that show the G418 resistance are recovered using a cloning ring. Culture of each clone thus recovered is continued in 10-cm plates until confluent.

After the cells have grown to confluency in a 6-cm dish, F protein expression is induced by infecting the cells with Adenovirus AxCANCre, for example, at MOI = 3, according to the method of Saito, *et al.* (Saito *et al.,* Nucl. Acids Res. 23: 3816-3821 (1995); Arai, T. *et al.,* J. Virol 72,1115-1121 (1998)).

### <3> Reconstitution and amplification of F defective Sendai virus (SeV)

The above-described plasmid pSeV18⁺/ΔF, into which a foreign gene has been inserted, is transfected to LLC-MK2 cells as follows: LLC-MK2 cells are seeded at 5x 10⁶ cells/dish in 100-mm dishes. When genomic RNA transcription is carried out with T7·RNA polymerase, cells are cultured for 24 hours, then infected at an MOI of about two for one hour at room temperature, with recombinant vaccinia virus expressing T7·RNA polymerase, which has been treated with psoralen and long-wave ultraviolet rays (365 nm) for 20 minutes (PLWUV-VacT7: Fuerst, T. R*. et al.,* Proc. Natl. Acad. Sci. USA 83, 8122-8126 (1986)). For the ultraviolet ray irradiation of vaccinia virus, for example, an UV Stratalinker 2400 equipped with five 15-watt bulbs can be used (catalogue No. 400676 (100V), Stratagene, La Jolla, CA, USA). The cells are washed with serum-free MEM, then an appropriate lipofection reagent is used to transfect the cells with a plasmid expressing the genomic RNA, and expression plasmids expressing the N, P, L, F, and HN proteins of paramyxovirus respectively. The ratio of amounts of these plasmids can be preferably set as 6:2:1:2:2:2, in this order, though is not limited thereto. For example, a genomic RNA-expressing plasmid as well as expression plasmids expressing the N, P, L, and F plus HN proteins (pGEM/NP, pGEM/P, pGEM/L, and pGEM/F-HN; WO00/70070, Kato, A*. et al.*, Genes Cells 1, 569-579 (1996)) are transfected at an amount ratio of 12 µg, 4 µg, 2 µg, 4 µg, and 4 µg/dish, respectively. After culturing for several hours, the cells are twice washed with serum-free MEM, and cultured in MEM comprising 40 µg/ml of cytosine β-D-arabinofuranoside (AraC: Sigma, St. Louis, MO) and 7.5 µg/ml of trypsin (Gibco-BRL, Rockville, MD). These cells are recovered, and the pellets are suspended in Opti-MEM (10⁷ cells/ml). Suspensions are freeze-thawed three times, mixed with lipofection reagent DOSPER (Boehringer Mannheim) (10⁶ cells/25 µl DOSPER), stood at room temperature for 15 minutes, transfected to the above-described cloned F-expressing helper cells (10⁶ cells/well in a 12-well-plate), and cultured in serum-free MEM (comprising 40 µg/ml AraC and 7.5 µg/ml trypsin) to recover the supernatant. Viruses defective in a gene other than F, for example, the HN or M gene, can also be prepared by similar methods to this.

When a viral gene-defective type vector is prepared, for example, if two or more different kinds of vectors, that comprise the different viral genes which are defective in the viral genome in the vectors, are transduced into the same cell, the viral proteins that are defective in each of the vectors are supplied by their expression from the other vectors. Thus, together, these vectors make up for protein deficiencies, and infectious virions can be formed. As a result, the replication cycle can amplify the viral vectors. In other words, when two or more kinds of vectors of this invention are inoculated in a combination that together supplements deficient viral proteins, mixtures of viral vectors defective in each of the viral genes can be produced on a large scale and at a low cost. When compared to viruses that are not deficient in viral genes, these viruses have smaller genomes, due to deficient viral genes, and can thus carry larger foreign genes. Further, these viruses, which lack proliferative ability due to deficient viral genes, are extracellularly attenuated, and maintaining coinfection is difficult. They are therefore sterilized, which is an advantage in environmental release management.

When, after administering a replicable paramyxoviral vector to an individual or cell, the proliferation of the viral vector must be restrained due to treatment completion and such, it is also possible to specifically restrain only the proliferation of the viral vector, with no damage to the host, by administering an RNA-dependent RNA polymerase inhibitor.

According to the methods of the present invention, the viral vectors of this invention can be released into the culture medium of virus-producing cells, for example, at a titer of 1x 10⁵ CIU/ml or more, preferably 1x 10⁶ CIU/ml or more, more preferably 5x 10⁶ CIU/ml or more, more preferably 1x 10⁷ CIU/ml or more, more preferably 5x 10⁷ CIU/ml or more, more preferably 1x 10⁸ CIU/ml or more, and more preferably 5x 10⁸ CIU/ml or more. Viral titers can be measured by a method described in this description and others (Kiyotani, K. *et al.,* Virology 177(1), 65-74 (1990); WO00/70070).

The recovered paramyxoviral vectors can be purified to become substantially pure. Purification can be performed by purification and separation methods known in the art, including filtration, centrifugal separation, and column purification, or any combinations thereof. "Substantially pure" means that a viral vector accounts for a major proportion of a sample in which the viral vector exists as a component. Typically, a substantially pure viral vector can be identified by confirming that the proportion of proteins derived from the viral vector is, for example, 10% or more of all of the proteins in a sample, preferably 20% or more, preferably 50% or more, preferably 70% or more, more preferably 80% or more, and further more preferably 90% or more (excluding, however, proteins and such added as carriers, stabilizers, etc.). Examples of specific methods for purifying paramyxoviruses are those that use cellulose sulfate ester or cross-linked polysaccharide sulfate ester (Japanese Patent Application Kokoku Publication No. (JP-B) Sho 62-30752 (examined, approved Japanese patent application published for opposition), JP-B Sho 62-33879, and JP-B Sho 62-30753), and those for methods for adsorbing them to polysaccharides comprising fucose sulfate and/or degradation products thereof (WO97/32010).

In preparing compositions comprising a vector, the vector can be combined with a pharmaceutically acceptable desired carrier or vehicle, as necessary. "A pharmaceutically acceptable carrier or vehicle" means a material that can be administered together with the vector which does not significantly inhibit gene transduction via the vector. For example, a vector can be appropriately diluted with physiological saline or phosphate-buffered saline (PBS) to form a composition. When a vector is grown in hen eggs or the like, the "pharmaceutically acceptable carrier or vehicle" may comprise allantoic fluids. Further, compositions comprising a vector may include carriers or vehicles such as deionized water and 5% dextrose aqueous solution. Furthermore, compositions may comprise, besides the above, plant oils, suspending agents, surfactants, stabilizers, biocides, and so on. The compositions can also comprise preservatives or other additives. The compositions comprising the vectors of this invention are useful as reagents and medicines.

Vector dose may vary depending upon the disorder, body weight, age, gender, and symptoms of patients, as well as purpose of administration, form of the composition to be administered, administration method, gene to be transduced, and so on; however, those skilled in the art can appropriately determine dosages. Administration route can be appropriately selected, although administration can be performed, for example, percutaneously, intranasally, perbronchially, intramuscularly, intraperitoneally, intravenously, intra-articularly, intraspinally, or subcutaneously, but is not limited to these routes. Administration can also be performed locally or systemically. Doses of the vector are preferably administered in a pharmaceutically acceptable carrier in a range of preferably about 10⁵ CIU/ml to about 10¹¹ CIU/ml, more preferably about 10⁷ CIU/ml to about 10⁹ CIU/ml, most preferably about 1x 10⁸ CIU/ml to about 5x 10⁸ CIU/ml. In humans, a single dose is preferably in the range of 2x 10⁵ CIU to 2x 10¹⁰ CIU, and can be administered once or more, so long as the side effects are within a clinically acceptable range. The same applies to the number of administrations per day. With animals other than humans, for example, the above-described doses can be converted based on the body weight ratio or volume ratio of a target site for administration (e.g. average values) between the objective animal and humans, and the converted doses can be administered to the animals. Subjects for administering compositions comprising the vectors of this invention include all mammals, such as humans, monkeys, mice, rats, rabbits, sheep, cattle, and dogs.

### Brief Description of the Drawings

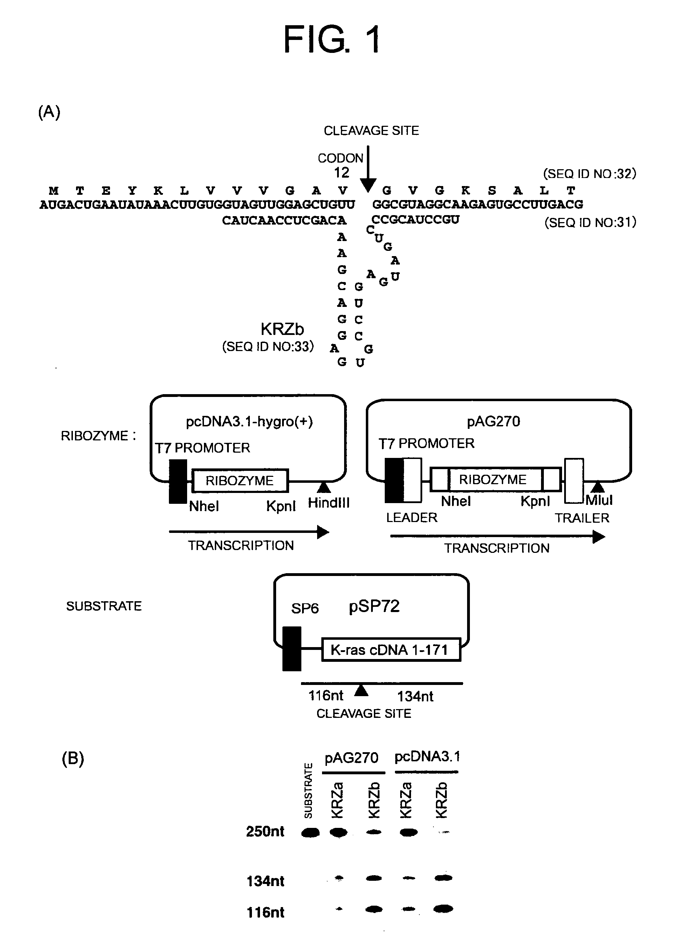

Fig. 1 is a diagram and photograph showing the structure (A) and activity (B) of a ribozyme that specifically cleaves K-ras. The nucleotide sequence of the target site and the amino acid sequence it encodes are shown in SEQ ID NOs: 31 and 32, respectively. The nucleotide sequence of the ribozyme is shown in SEQ ID NO: 33.

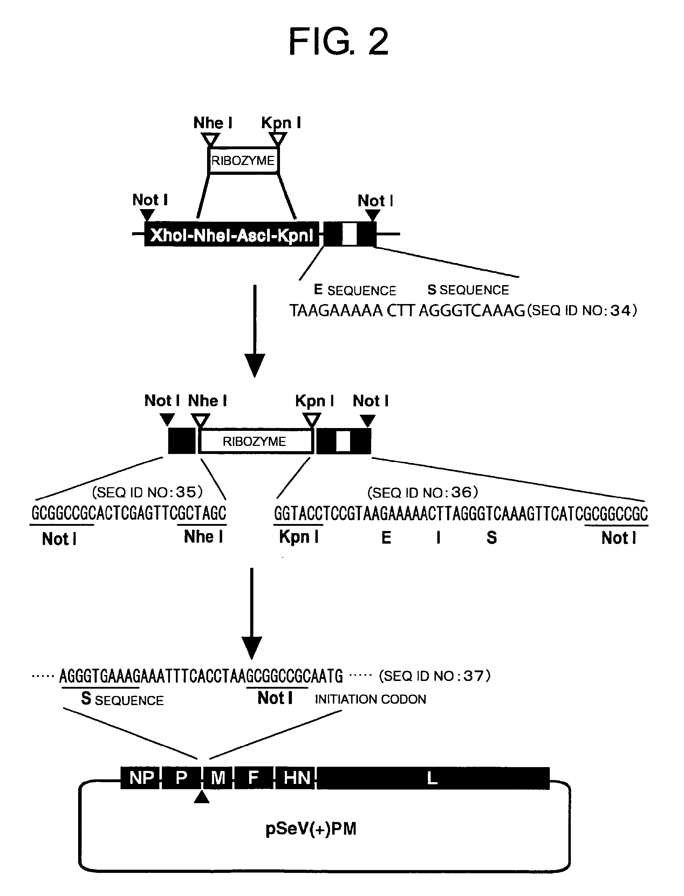

Fig. 2 is a schematic diagram showing the construction of a Sendai viral vector that encodes a ribozyme that specifically cleaves K-ras. The sequences of the nucleotides attached to each end of the ribozyme in the vector are shown in SEQ ID NOs: 35 and 36. The EIS sequence is shown in SEQ ID NO: 34. The nucleotide sequence in the vicinity of the cloning site of pSeV(+)PM is shown in SEQ ID NO: 37.

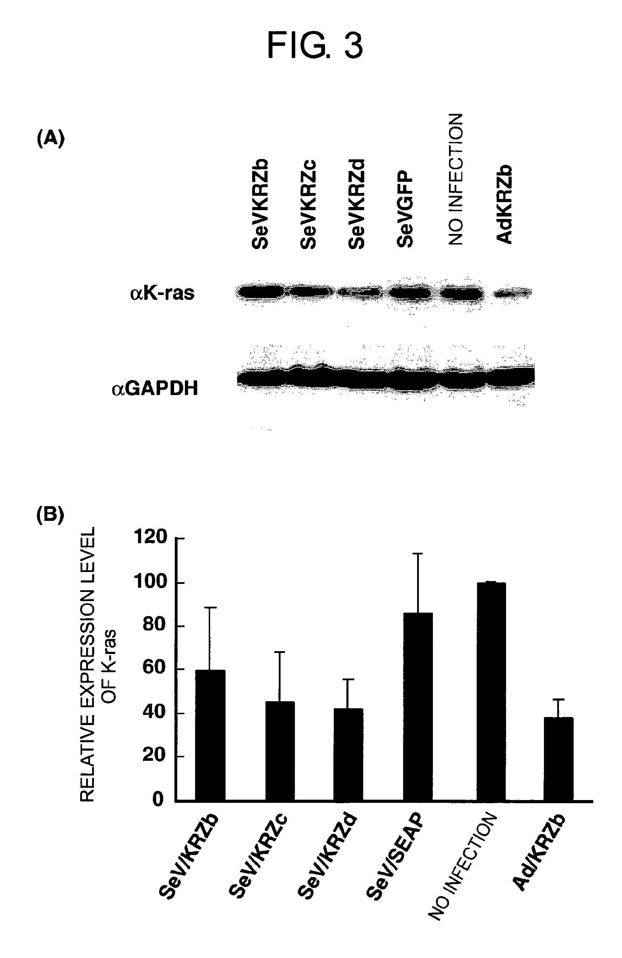

Fig. 3 is a photograph and diagram showing the activity of a Sendai viral vector that encodes a ribozyme that specifically cleaves K-ras.

### Best Mode for Carrying Out the Invention

Hereinafter, the present invention will be explained in more detail with reference to Examples, but is not to be construed as being limited thereto. All the references cited herein have been incorporated as parts of this description.

### [Example 1] Plasmid construction

Table 1 shows synthetic oligonucleotides that encode ribozymes. The target selected was the mutated valine at position 12 in the mutant V12 K-ras. There have already been reports of ribozymes targeted to this site. In this invention, KRZa and KRZb (Fig. 1A, upper panel), of Table 1 were prepared with reference to the designs of Tokunaga *et al.* and Zhang *et al.,* (Tokunaga, T. *et al.* Br J Cancer 83(6), 833-9 (2000); Zhang, Y.A. *et al.* Gene Ther 7(23), 2041-50 (2000)), and Funato *et al.,* (Funato, T. *et al.* Cancer Gene Ther 7(3), 495-500 (2000)), respectively. KRZc and KRZd, whose recognition sites are a different size to those of the above, were designed in the present invention. Their respective sequences are: KRZa 
(sense: 5'-CTAGCTACGCCCTGATGAGTCCGTGAGGACGAAACAGCTGGTAC-3'/SEQ ID
NO: 17, antisense: 5'-CAGCTGTTTCGTCCTCACGGACTCATCAGGGCGTAG-3'/SEQ ID
NO: 18);
 KRZb 
(sense:
5'-TGCCTACGCCCTGATGAGTCCGTGAGGACGAAACAGCTCCAACTAGG-3'/SEQ ID
NO:19, antisense:
 
5'-CTAGTTGGAGCTGTTTCGTCCTCACGGACTCATCAGGGCGTAGGCAGG-3'/SEQ ID
NO: 20);
 KRZc 
(5'-CTAGCCCACTCTTGCCTACGCCCTGATGAGTCCGTGAGGACGAAACAGCTCCAAC
TACCACAAGGTAC-3'/SEQ ID NO: 21, sntisense:
 
5'-CTTGTGGTAGTTGGAGCTGTTCGTCCTCACGGACTCATCAGGGCGTAGGCAAGAG
TGGG-3'/SEQ ID NO: 22);
 KRZd 
(sense:
5'-CTAGCCTCAAGGCACTCTTGCCTACGCCCTGATGAGTCCGTGAGGACGAAACAGCT
CCAACTACCACAAGTTTATGGTAC-3'/SEQ ID NO: 23, antisense:
 
5'-CATAAACTTGTGGTAGTTGGAGCTGTTTCGTCCTCACGGACTCATCAGGGCGTAGG
CAAGAGTGCCTTGAGG-3'/SEQ ID NO: 24).
 A *Nhe*I site was added to one end and a *Kpn*I site was added to the other end of each sequence described above. The sense and antisense strands were mixed at a ratio of 1:1 and allowed to anneal. Each sequence was subcloned into pcDNA3.1-hygro(+), pAG270 containing the mini-genome of Sendai virus, and pSeV(+)PM (Tokusumi, T. *et al.* Virus Res 86(1-2), 33-8 (2002)) (Fig. 1A, middle panel). The pAG270 containing a ribozyme was constructed by annealing 
5'-GGCCGCGCTAGCTTGCTCGAGCAAGGCGCGCCTCCGTAAGAAAAACTTAGGGTGA
AAGTTCATCGC-3' (SEQ ID NO: 25) and
5'-GGCCGCGATGAACTTTCACCCTAAGTTTTTCTTACGGAGGCGCGCCTTGCTCGAGC
AAGCTAGCGC-3' (SEQ ID NO: 26),
 ligating the resultant duplex into the *Not*I site of pSeV18⁺ (Hasan, M. K*. et al.,* J. General Virology 78: 2813-2820 (1997)), digesting the plasmid with *Nhe*I and *Kpn*I to remove most of the central portion in the viral genome, and ligating the synthetic DNA encoding the ribozyme into that site. A substrate to test the activity of the ribozyme *in vitro* was prepared by using PCR to amplify the region covering nucleotides 1 to 171 of K-ras cDNA, and inserting it into pCRII (Invitrogen) using the TA cloning method (the bottom panel in Fig.1A). The primers used are: 5'-ATGAGGGACCAGTACATGAGGA-3' (SEQ ID NO: 27) and 5'-GTCGAGAATATCCAAGAGACAGGTTTCTCC-3' (SEQ ID NO: 28).

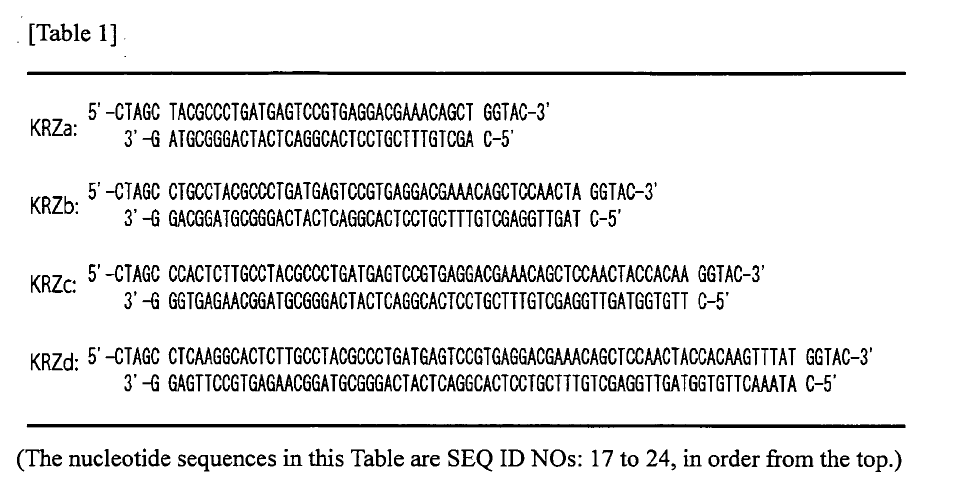

### [Example 2] In-vitro cleavage assay

Ribosomal activity was tested to determine whether it was influenced by the transcription termination sequence, intervening sequence, and transcription initiation sequence, which are specific to paramyxovirus, such as SeV. pCRII/K-ras171 was linearized and transcribed in the presence of [α-³²P]UTP using SP6 RNA polymerase to prepare ³²P-labeled RNA substrate. The substrate was purified by electrophoresis using a 6% polyacrylamide gel containing 8 M urea. Meanwhile, the ribozyme was transcribed from the linearized plasmid using T7 RNA polymerase. 10 µg of ribozyme was combined with approximately 5000 cpm of the ³²P-labeled RNA substrate in a cleavage buffer (40 mM Tris-HCl (pH7.5), 20 mM MgCl₂, 2 mM spermidine, and 10 mM DTT) and incubated at 37°C overnight. This was electrophoresed in a 6% polyacrylamide gel containing 8 M urea, and the gel was analyzed in BAS2000. Fig. 1B shows the results obtained. The activity of the ribozyme is only slightly lower than that of a ribozyme containing no paramyxovirus-specific sequences, and the sequences were thus judged to have almost no influence on the activity. Meanwhile, the activity of KRZa was low, regardless of whether or not the paramyxovirus-specific sequences were present. It was thus decided that the sequences were not carried by the SeV vector.

### [Example 3] Preparation of recombinant SeV vector

The SeV vector was prepared basically by the method described in the references (Tokusumi, T. *et al.* Virus Res 86(1-2), 33-8 (2002)). The multi-cloning site of pAC116 was modified to introduce a *Nhe*I*-Kpn*I site. The 5'-cloning site comprises a 72-mer fragment flanked by *Not*I sites (5'-GGCCGCACTCGAGTTCGCTAGCTTGGCGCGCCGGTACCTCCGTAAGAAAAACTTA GGGTCAAAGTTCATCGC-3'/SEQ ID NO: 29) and contains an *Nhe*I site, *Kpn*I site, and EIS sequence. The DNA encoding the ribozyme was subcloned into the *Nhe*I*lKpn*I site. The DNA was excised using *Not*I and subcloned into the *Not*I site of pSeV(+)PM (Tokusumi, T. *et al.* Virus Res 86(1-2), 33-8 (2002)) (Fig. 2). The sequence in the vicinity of the *Not*I site of pSeV(+)PM is: 5'-AGGGTGAAAGAAATTTCACCTAAGCGGCCGCAATG-3' (SEQ ID NO: 30; from the left the portions underlined correspond to the S sequence, *Not*I site, and initiation codon). The construct was transfected to LLC-MK2 under infection with the vaccinia virus VacT7 expressing T7 RNA polymerase. After three days, the cells were harvested and lysed by three cycles of freeze-thawing to prepare a lysate. The resulting lysate was inoculated to 10-day-old embryonated hen eggs for viral expansion.

### [Example 4] Western blotting

The recombinant SeV was allowed to infect to SW480, a human colorectal cancer cell line, at an MOI of 10. After two days, the cells were harvested. The cells were lysed in an SDS-PAGE sample buffer and heated at 100°C. Then, the sample was fractionated by SDS-PAGE using 10 to 20% gel and transferred onto PVDF membrane (Millipore). The membrane was analyzed by Western blotting using human K-ras antibody (Oncogene) and anti-GAPDH antibody (TREVIGEN). The detection was carried out using ECL Plus (Amersham). The signals were analyzed and quantified using the image analyzer LAS 1000 (Fig. 3). The band intensities were determined by the image analyzer and normalized using the intensity determined using anti-GAPDH antibody. As a result, every Sendai virus that encoded a ribozyme was found to suppress the expression of endogenous K-ras. Thus, SeV allows the expression of active ribozymes. The shortest ribozyme exhibited only low activity; however, as vectors containing longer ribozyme were used, the activity was greater. The greatest activity was from the SeV containing KRZc comprising the longest recognition site.

### Industrial Applicability

The present invention provides paramyxoviral vectors that allow the expression of ribozymes. The vectors of the present invention can suppress the expression of desired target genes in cells. The paramyxoviral vectors can be used to introduce genes into a broad range of cell types, and thus are suitable as gene therapy vectors to be introduced into the body *in vivo* or *ex vivo.* In particular, vectors encoding ribozymes that suppress the expression of genes that cause diseases can be used in gene therapy for those diseases.

## Claims

1. A paramyxoviral vector encoding RNA having a catalytic activity.

2. The paramyxoviral vector of claim 1, wherein the length of the RNA is 50 nucleotides or more.

3. The paramyxoviral vector of claim 1, wherein the length of the RNA is 60 nucleotides or more.

4. The paramyxoviral vector of claim 1, wherein the RNA is a hammerhead ribozyme.

5. The paramyxoviral vector of claim 1, wherein the paramyxovirus is Sendai virus.
